# EUROPEAN PATENT APPLICATION

(11) **EP 3 646 916 A1**
(43) Date of publication of application: **06.05.2020**
(21) Application number: 18824635.9
(22) Date of filing: 25.06.2018
(51) Int. Cl.: A61M 25/00, A61B 17/22, A61B 17/3207, A61M 25/09

(54) **CATHETER, SEPARATOR, AND SUCTION SYSTEM**

(30) Priority: 27.06.2017 JP 2017125178; 31.05.2018 JP 2018104347
(71) Applicant: Nipro Corporation, Osaka-shi, Osaka 531-8510 (JP)
(72) Inventor: MATSUMOTO, Kuniaki, Osaka-shi Osaka 531-8510 (JP)
(74) Representative: Betten & Resch
(86) International application number: PCT/JP2018/023923
(87) International publication number: WO 2019/004100

(57) **Abstract**

A catheter 50 having an elongated lumen LM extending along a longitudinal axis, includes: an inner layer member 61 having an inner circumferential surface 61a in contact with the lumen LM; an outer layer member 71 having an outer circumferential surface 62a of the catheter 50; a first reinforcing member 71 having a shape of coil, provided outside the inner layer member 61, between the inner circumferential surface 61a and the outer circumferential surface 62a; and a second reinforcing member 72 having a shape of braid, provided outside the first reinforcing member 71, between the inner circumferential surface 61a and the outer circumferential surface 62a. According to this technique, rigidity, pushability, torque transmission performance, and kink resistance of the catheter can be ensured while the thickness of the catheter is kept small.

## Description

### [TECHNICAL FIELD]

The present invention relates to a catheter which can be introduced into a body percutaneously. The present invention also relates to a separator for mechanically removing foreign substances, such as thrombus, present in a blood vessel. The present invention also relates to a suction system for removing foreign substances, such as thrombus, present in a blood vessel.

### [BACKGROUND]

There is known a method of dissolving a foreign substance using a drug when a cerebral blood vessel (cerebral main artery) is clogged with a foreign substance, such as thrombus, thereby developing cerebral infarction (acute cerebral artery occlusion). However, depending on a patient, this drug may not work effectively, or the drug cannot be administered. In that case, a method is used in which a catheter is percutaneously introduced into the body, and then a flexible core wire, called separator, is inserted through the catheter while the separator moves to mechanically crush or entwine with the foreign substance, which can be removed from inside the blood vessel to outside the body by suction or entwined away with a stent retriever (i.e., stent-type thrombus retrieving device) to reopen the cerebral blood vessel.

The catheter needs to be inserted into the blood vessel in an arm, leg, or the like to ensure that a tip end of the catheter reaches a treatment site. Thus, the catheter is required to have various properties, such as pushability, torque performance, flexible bendability, restorability, and kink resistance. In the following Patent Documents a catheter with a reinforcing structure for realizing the above properties is disclosed.

Patent Document 6 discloses, for example, a separator including a coil as a base and a separation element made of resin. The separation element has a conical shape and is solid. The separation element is also adhered to the coil. Foreign substances crushed by the separation element are sucked using a pump and removed from inside a blood vessel to outside a body.

### [PRIOR ART DOCUMENT]

### [PATENT DOCUMENT]

[Patent Document 1] JP 2017-189213 A
[Patent Document 2] JP 2005-296078 A
[Patent Document 3] JP H10-57495 A
[Patent Document 4] JP 5659788 B2
[Patent Document 5] JP 2010-88833 A
[Patent Document 6] WO 2006/031410 A

### [SUMMARY OF THE INVENTION]

### [PROBLEM TO BE SOLVED BY THE INVENTION]

The tip end of the catheter, which precedes inside the blood vessel having a thin and complicated shape, requires flexibility, restorability, and the like. On the other hand, the base end of the catheter, which transmits operation performed by an operator to the entire catheter, requires relatively high rigidity, pushability, torque transmission performance, kink resistance, and the like. In addition, in order to put a medical device/instrument in and out through a lumen of the catheter and to maintain a force for sucking the thrombus, a larger inner diameter of the catheter is preferable. However, an outer diameter of the catheter is limited to a certain extent in order to secure insertability into the blood vessel and the like and operability within the blood vessel and the like. Therefore, as the inner diameter of the catheter increases, a thickness of the catheter decreases, thereby reducing the strength thereof. As a result, pushability, torque transmission performance, and kink resistance are degraded.

Generally, a thrombus crushing member of the separator needs to have a structure that can suitably crush a foreign substance, such as thrombus. In addition, the thrombus crushing member of the separator needs to be firmly fixed to the base so as not to be separated from the base even if the member receives a force from a wall of the blood vessel, the foreign substance, and the like. However, in the separator disclosed in Patent Document 6, the thrombus crushing member is attached using an adhesive to the coil, in which there is room for improvement in terms of fixation strength. Further, the thrombus crushing member of the separator disclosed in Patent Document 6 is the solid member made of resin, hence, it may be difficult for an operator to operate the separator, particularly at a curved portion of the blood vessel. Therefore, improvement in the operability of the separator is also desired to prevent the operator from erroneously operating the separator and damaging the blood vessel.

It is an object of the present invention to provide a catheter having a novel structure that can ensure rigidity, pushability, torque transmission performance, and kink resistance of the catheter while the thickness of the catheter is kept small.

Further, it is an object of the present invention to provide a suction system using the above catheter.

Further, it is an object of the present invention to provide a separator in which a thrombus crushing member is firmly fixed to produce excellent operability.

Further, it is an object of the present invention to provide a suction system using the above separator.

### [MEANS FOR SOLVING THE PROBLEM]

In order to achieve the above-mentioned object, a catheter according to the present invention has an elongated lumen extending along a longitudinal axis, the catheter including:
an inner layer member having an inner circumferential surface in contact with the lumen;
an outer layer member having an outer circumferential surface of the catheter;
a first reinforcing member having a shape of coil, provided outside the inner layer member, between the inner circumferential surface and the outer circumferential surface; and
a second reinforcing member having a shape of braid, provided outside the first reinforcing member, between the inner circumferential surface and the outer circumferential surface.

According to the present invention, between the inner circumferential surface and the outer circumferential surface of the catheter, not only the first reinforcing member having a shape of coil but also the second reinforcing member having a shape of braid is provided. When the catheter is curved, a coil gap on the inner side of the curved portion becomes smaller and the outer layer member is compressed, while the coil gap on the outer side of the curved portion becomes larger and the outer layer member is stretched. When the catheter is further curved, the outer layer member may be further stretched, resulting in breakage. As a countermeasure therefor, a second reinforcing member having a shape of braid is provided outside the first reinforcing member so as to support stress generated on the outer side of the curved portion, thereby preventing the outer layer member from being broken. As a result, even when the thickness of the catheter is small, the strength can be maintained to ensure pushability, torque transmission performance and kink resistance.

It is preferable in the present invention that the second reinforcing member is constituted of an elongated member made of synthetic resin.

According to this aspect, a synthetic resin that is softer and easier to process can used to realize a flat reinforcing member having a small thickness. This can further reduce the thickness of the catheter while maintaining the strength.

It is preferable in the present invention that the elongated member is constituted of a bundle of plural fibers.

According to this aspect, rigidity and flexibility of the catheter can be adjusted by increasing or decreasing the number of fibers. More specifically, decreasing the number of fibers leads to higher flexibility, or increasing the number of fibers leads to higher rigidity. Thus, the catheter having desired performance can be easily realized by increasing or decreasing the number of fibers. In addition, since the elongated member is constituted of plural fibers, even if a part of the elongated member is broken, that is, even if a part of the plural fibers is broken, kinking of the catheter does not occur immediately, and therefore, the catheter having excellent durability can be obtained.

It is preferable in the present invention that the first reinforcing member is constituted of a wire having a rectangular cross section.

According to this aspect, the catheter can have an increased strength per unit length.

It is preferable in the present invention that the first reinforcing member is constituted of a metal wire.

According to this aspect, the first reinforcing member can have a high strength to support the stress generated at the curved portion of the catheter.

It is preferable in the present invention that the second reinforcing member is provided in a region at a predetermined distance from the proximal end.

According to this aspect, there is a region where the second reinforcing member is present and there is another region where the second reinforcing member is absent, thereby realizing the catheter having different properties for each region.

Further, a suction system according to the present invention includes:
the above-described catheter; and
a suction pump connected to the catheter.

According to the present invention, the catheter can be used which has a large inner diameter that can maintain a high suction force and a small outer diameter excellent in terms of insertability into the blood vessel and the like and operability in the blood vessel and the like, as well as sufficient strength. Therefore, foreign substances, such as thrombus, present in the blood vessel can be efficiently removed.

In addition, a separator according to the present invention includes:
a core wire having a longitudinal axis; and
a coil element wound around the longitudinal axis of the core wire and having a distal end and a proximal end joined to the core wire;
wherein the coil element includes a first coil unit, a second coil unit having a first end and a second end connected to the first coil unit, and a distal joint part and a proximal joint part provided at the first end and the second end, respectively, and
the second coil unit has a bulging part having an outer diameter larger than outer diameters of the first end and the second end.

### [EFFECT OF THE INVENTION]

According to the present invention, it can provide a catheter having a novel structure that can ensure rigidity, pushability, torque transmission performance, and kink resistance of the catheter while the thickness of the catheter is kept small. In addition, it can provide a suction system using the above catheter.

Further, according to the present invention, the bulging part provided in the second coil unit of the coil element can mainly functions as a thrombus crushing member. The coil element includes a distal joint part and a proximal joint part provided at the first end and the second end of the second coil unit, respectively, to realize a separator in which the thrombus crushing member is firmly fixed. Furthermore, the bulging part is constituted of a coil to realize a separator which is excellent in operability at a curved portion of the blood vessel.

### [BRIEF DESCRIPTION OF THE DRAWINGS]

Fig. 1 is a side view showing an embodiment of a catheter according to the present invention.
Fig. 2 is a partial side view of a base end section of the catheter shown in Fig. 1.
Fig. 3 is a cross-sectional view taken along a line A-A of Fig. 1.
Fig. 4 is a longitudinal sectional view taken along a longitudinal axis, corresponding to the partial side view of Fig. 2.
Fig. 5 is a side view showing another embodiment of the catheter according to the present invention.
Fig. 6 is a side view showing a distal end of a separator according to an embodiment of the present invention.
Fig. 7 is a cross-sectional view showing the distal end of the separator according to the embodiment of the present invention.
Fig. 8 is a view showing a suction system provided with a separator according to the embodiment of the present invention.
Fig. 9 is a view showing an operation of the suction system of Fig. 8.
Fig. 10 is a view showing an operation of the suction system of Fig. 8.
Fig. 11 is a view showing an operation of the suction system of Fig. 8.
Fig. 12 is a cross-sectional view showing a distal end of a separator according to another embodiment of the present invention.
Fig. 13 is a side view showing the distal end of a separator according to another embodiment of the present invention.
Fig. 14 is a side view showing the distal end of a separator according to another embodiment of the present invention.

### [EMBODIMENT FOR CARRYING OUT THE INVENTION]

Hereinafter, embodiments of the present invention will be specifically described with reference to the drawings.

### [1. Catheter]

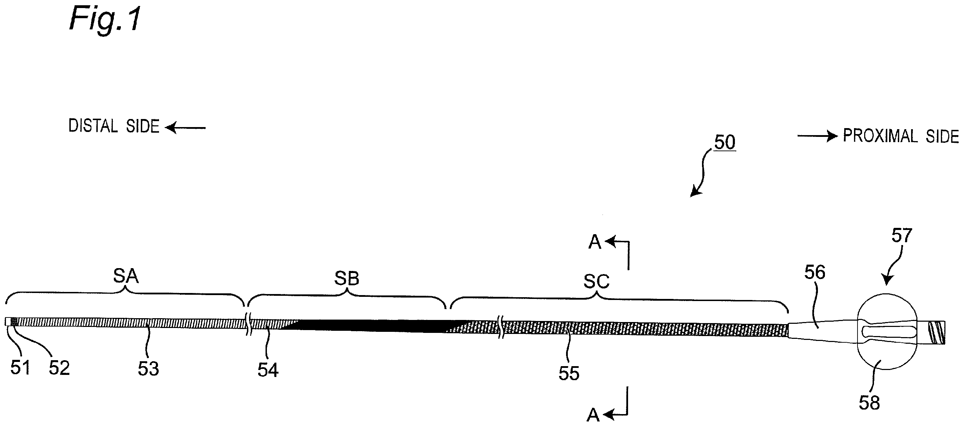

Fig. 1 is a side view showing an embodiment of a catheter according to the present invention. A catheter 50 is a hollow member having an elongated lumen extending along a longitudinal axis. For easy understanding, the catheter 50 is generally divided into a tip end section SA, an intermediate section SB, and a base end section SC, where geometric shape, such as outer diameter and inner diameter, and physical properties, such as elasticity and restorability, are designed to change in a continuous and/or stepwise manner from a proximal end of the base end section SC toward a distal end of the tip end section SA. For example, the outer diameter of the catheter 50 may change in a range of 1.7 mm to 2.2 mm, and the inner diameter may change in a range of 1.5 mm to 1.8 mm. The tip end section SA, the intermediate section SB and the base end section SC may be further divided into plural regions each having different geometric shapes and physical properties. The geometrical shapes and physical properties may be also made uniform from the proximal end of the base end section SC toward the distal end of the tip end section SA.

The tip end section SA is set in a region at a predetermined distance from the distal end and includes a tube 53 having an elongated lumen extending along the longitudinal axis. A tip 51 made of flexible material friendly to blood vessels is attached to the distal end of the tube 53. An X-ray contrast marker 52, such as Pt (platinum) alloy, is attached in the vicinity of the tip 51.

The base end section SC is set in a region at a predetermined distance from the proximal end and includes a tube 55 having an elongated lumen extending along the longitudinal axis. The intermediate section SB is set in a region between the tip end section SA and the base end section SC and includes a tube 54 having an elongated lumen extending along the longitudinal axis. The tubes 53, 54, 55 may be connected with each other using a well-known joining technique, such as welding, gluing, crimping, melting, or the like, or may be integrally formed.

A hub 57 having radially extending fins 58 is connected to the proximal end of the tube 55. The hub 57 is a hollow member and in fluid communication with the tubes 53, 54, 55. On a distal side of the hub 57, a strain relief 56 for preventing kinking is mounted so as to cover the tube 55.

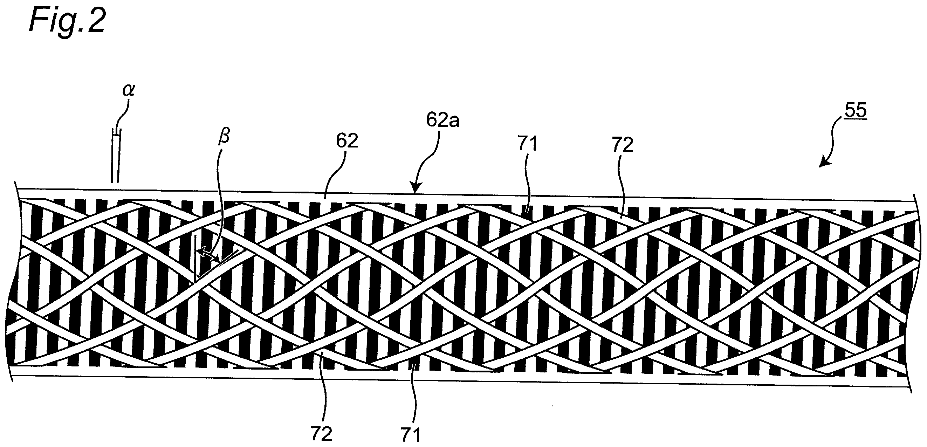

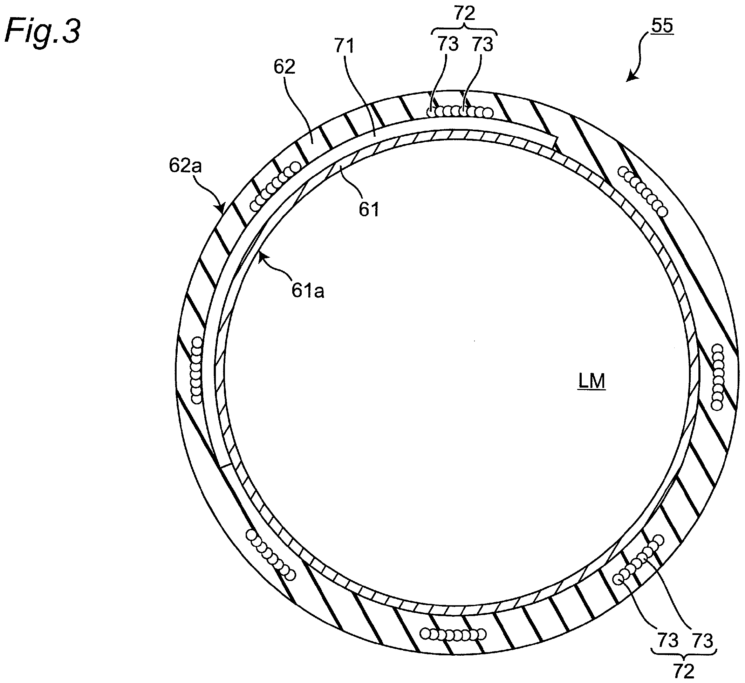

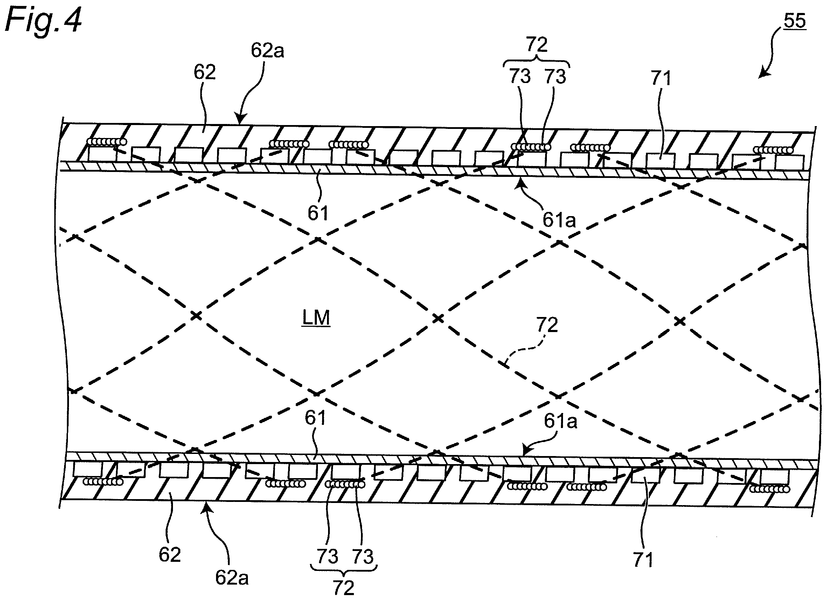

Fig. 2 is a partial side view of the base end section SC of the catheter 50 shown in FIG. 1. Fig. 3 is a cross-sectional view taken along a line A-A of Fig. 1. Fig. 4 is a longitudinal sectional view taken along the longitudinal axis, corresponding to the partial side view of Fig. 2.

The tube 55 includes an inner layer member 61, an outer layer member 62, a first reinforcing member 71, and a second reinforcing member 72. The inner layer member 61 has an inner circumferential surface 61a in contact with a lumen LM. The outer layer member 62 has an outer circumferential surface 62a of the catheter 50. The first reinforcing member 71 is provided between the inner circumferential surface 61a and the outer circumferential surface 62a and outside the inner layer member 61. The second reinforcing member 72 is provided between the inner circumferential surface 61a and the outer circumferential surface 62a and outside the first reinforcing member 71.

The inner layer member 61 can be preferably formed of a fluorine-based thermoplastic synthetic resin, for example, polytetrafluoroethylene (PTFE), polyvinylidene fluoride (PVDF), perfluoroalkoxy fluorine resin (PFA), and more preferably formed of polytetrafluoroethylene (PTFE). The inner layer member 61 is exemplified here as a single layer tube, but may be a multilayer tube made of the same or different materials.

The outer layer member 62 can be preferably formed of a thermoplastic synthetic resin, for example, polyether block amide (PEBA), polyethylene (PE), polypropylene (PP), polyamide (PA), polyimide (PI), polyamide imide (PAI), polyethylene terephthalate (PET), polyurethane (PU), nylon elastomer, ethylene-vinyl acetate resin (EVA), polyvinyl chloride (PVC), and more preferably formed of polyamide (PA). On the outer circumferential surface 62a of the outer layer member 62, a hydrophilic coat, such as hyaluronic acid, polyvinyl pyrrolidone, polyvinyl alcohol (PVA), is preferably applied. The outer layer member 62 is exemplified here as a single layer tube, but may be a multilayer tube made of the same or different materials.

The first reinforcing member 71 is formed so as to have a shape of coil. In other words, the first reinforcing member 71 has a structure in which a wire is helically wound clockwise or counterclockwise along the longitudinal axis. A helical lead angle α (angle of inclination of a helix relative to a vertical plane of the longitudinal axis) of the first reinforcing member 71 is set in a range of 0° < α < 90°, preferably 0° < α ≤ 15°, and more preferably 0° < α ≤ 10°. The lead angle α may vary along the longitudinal axis, and for example, may increase continuously and/or stepwise from the proximal end to the distal end of the catheter.

The first reinforcing member 71 can be preferably constituted of a metal wire, for example, a wire made of stainless steel (SUS) and Ni-Ti (nickel-titanium) alloy. With this configuration, when the catheter is curved, stress generated on the outer side of the curved portion can be supported by the first reinforcing member 71, and accordingly, deformation of the outer layer member 62 can be suppressed.

A cross-sectional shape of the first reinforcing member 71 may be a perfect circle, ellipse, square, rectangle, polygon, or the like, and preferably, a wire having a rectangular cross section, so-called flat wire, can be used, thereby enhancing strength per unit length.

The second reinforcing member 72 is formed so as to have a shape of braid. In other words, the second reinforcing member 72 constitutes a tubular braided body in which a first group having plural (for example, 4 to 8) elongated members arranged at equal intervals around the longitudinal axis and helically wound clockwise along the longitudinal axis, and a second group having plural (for example, 4 to 8) elongated members arranged at equal intervals around the longitudinal axis and helically wound counterclockwise along the longitudinal axis, are braided to be alternately overlapped with each other. The above elongated member may be hollow or solid, and as long as the tubular braided body can be formed, a length in a lateral direction of the elongated member may be freely selected and the elongated member may have a linear or strip shape.

A helical lead angle β (angle of inclination of a helix relative to the vertical plane of the longitudinal axis) of the second reinforcing member 72 is set in a range of 0° < β ≤ 90°, preferably 15° ≤ β ≤ 75°, and more preferably 30° ≤ β ≤ 60°. In this case it is preferable that the lead angle β of the second reinforcing member 72 is larger than the lead angle α of the first reinforcing member 71, that is, satisfy α < β. With this configuration, both of the longitudinal component and the circumferential component of the stress generated in the catheter can be supported. The lead angle β may vary along the longitudinal axis, and for example, may increase continuously and/or stepwise from the proximal end to the distal end of the catheter.

The second reinforcing member 72 can be preferably formed of an elongated member made of synthetic resin, for example, an elongated member made of nylon resin, polyethylene resin, polyetheretherketone (PEEK), polysulfone (PSF), polyphenylene sulfide (PPS), and polybutylene terephthalate (PBT), an elongated member made of metal, for example, an elongated member made of stainless steel (SUS) and Ni-Ti alloy, or an elongated member made of carbon fiber, glass fiber, or the like, and more preferably, formed of an elongated member made of nylon resin. With this configuration, when the catheter is curved, the stress generated on the outer side of the curved portion can be supported by both the first reinforcing member 71 and the second reinforcing member 72, thus, deformation and breakage of the outer layer member 62 can be suppressed.

One elongated member constituting the second reinforcing member 72 is preferably constituted of a bundle of plural fibers 73, and with this configuration, a flat and thin reinforcing member having a high strength can be realized. Although Figs. 3 and 4 illustrate the case where one elongated member is constituted of a bundle of eight fibers 73, the elongated member can be constituted of a bundle of two to seven, or nine or more fibers 73. Moreover, although Figs. 3 and 4 exemplify the case where the plural fibers 73 are arranged in a line so as to be adjacent to each other in the circumferential direction, the fibers may be arranged to have a cross section in shape of perfect circle, ellipse, square, rectangle or polygon. The above fibers may be hollow or solid.

Next, a method of manufacturing the catheter will be illustrated below, however, the method of manufacturing the catheter is not limited thereto. First, a tubular inner layer member 61 is provided. Next, the first reinforcing member 71 is wound around the outer circumferential surface of the inner layer member 61 in shape of coil. Thereafter, the second reinforcing member 72 is braided over the outer circumferential surface of the first reinforcing member 71 in shape of braid. Then, a material for the outer layer member 62 is applied to the outer circumferential surface and then cured to form the outer layer member 62. Thus, the tube 55 constituting the base end section SC is obtained.

The above-mentioned embodiment has exemplified a structure where the first reinforcing member 71 is made in contact with the outer circumferential surface of the inner layer member 61, however, the first reinforcing member 71 may be separated from the outer circumferential surface of the inner layer member 61, or may be partially embedded inside the inner layer member 61.

Moreover, the above-mentioned embodiment has exemplified a structure where the second reinforcing member 72 is made in contact with the outer circumferential surface of the first reinforcing member 71, however, the second reinforcing member 72 may be separated from the outer circumferential surface of the first reinforcing member 71.

The tube 53 constituting the tip end section SA and the tube 54 constituting the intermediate section SB have a structure where the second reinforcing member 72 is omitted in the structure of the tube 55. A cross-sectional shape of the first reinforcing member 71 in the tubes 53 and 54 may be a perfect circle, ellipse, square, rectangle, polygon, or the like, and preferably, a wire having a perfect circular cross section, so-called round wire, can be used, thereby enhancing flexibility per unit length.

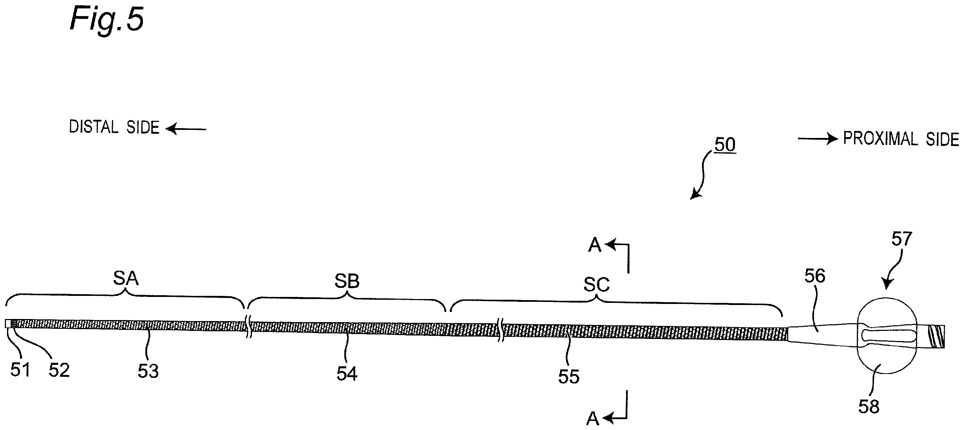

Fig. 5 is a side view showing another embodiment of the catheter according to the present invention. The tube 53 constituting the tip end section SA and the tube 54 constituting the intermediate section SB also have such a structure as shown in Figs. 2 to 4 and, and the second reinforcing member 72 having a shape of braid is provided similarly to the tube 55 constituting the base end section SC. With this configuration, rigidity, pushability, torque transmission performance, and kink resistance can be ensured throughout the catheter.

Although the above-mentioned embodiment has exemplified the case where the catheter 50 is of single lumen type, the present invention is applicable also to a double lumen type or triple lumen type catheter.

Further, although the above-mentioned embodiment has exemplified the case where the catheter 50 is of over-the-wire type, the present invention is applicable also to a rapid exchange type catheter having a lumen for guide wire provided independently.

Still further, although the above-mentioned embodiment has exemplified the case where the catheter 50 is introduced into the blood vessel percutaneously, the present invention is applicable to a catheter that can be introduced into a body cavity, such as thoracic cavity and abdominal cavity, a digestive tract, such as mouth and anus, and a ureter.

### [2. Suction system]

A suction system according to the present invention includes the above-mentioned catheter 50 and a suction pump connected to the catheter 50.

When the above suction system is used, an operator operates the catheter 50 while confirming an X-ray contrast marker provided at the catheter 50 during radiographic visualization. The operator advances and positions the catheter 50, for example, from the femoral artery toward a thrombus occlusion site in the cerebral blood vessel. Next, when the operator operates the suction pump, the thrombus moves through the catheter 50 and is removed outside of the body. In addition, the operator may use a separator or stent retriever in combination to perform an operation of crushing or entwining with the thrombus.

### [3. Separator]

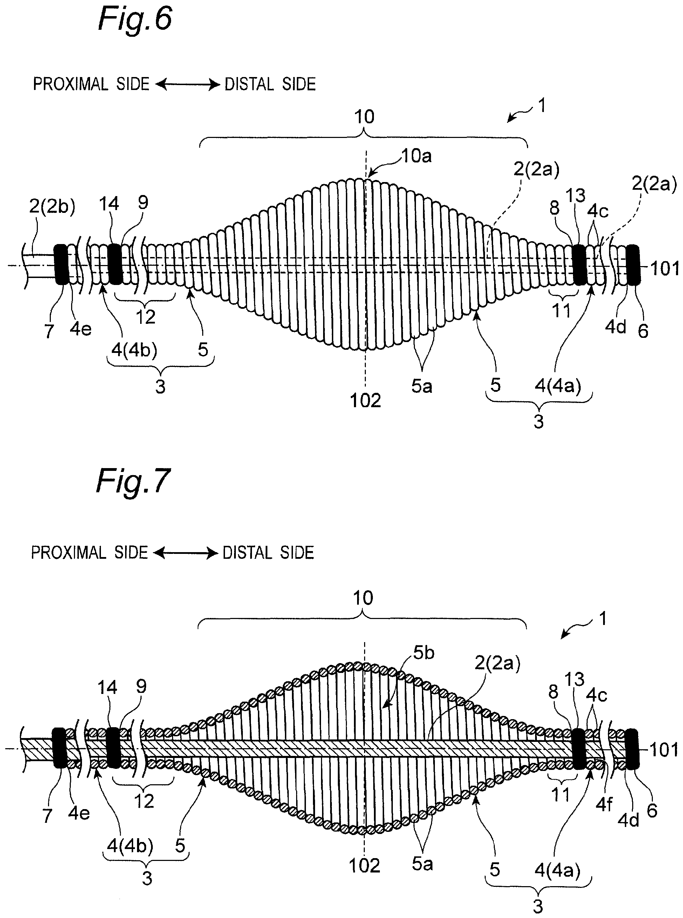

Fig. 6 is a side view showing a distal end of a separator 1 according to an embodiment of the present invention, and Fig. 7 is a cross-sectional view showing the distal end of the separator 1. The separator 1 generally has an outer diameter sufficiently smaller than an inner diameter of a cerebral blood vessel. As described later, the separator 1 is inserted into a catheter connected to a suction pump, and is used to mechanically crush or entwine with a foreign substance, such as thrombus, present in the cerebral blood vessel and remove the foreign substance outside the body. The separator 1 is elongated and includes a core wire 2 and a coil element 3 provided at a distal end of the core wire 2. The core wire 2 and the coil element 3 constituting the separator 1 are flexible.

The core wire 2 has a longitudinal axis 101. Hereinafter, a direction in which the longitudinal axis 101 of the core wire 2 extends is referred to as a longitudinal direction of the separator 1. In the longitudinal direction, a side on which the coil element 3 is provided in the separator 1 is referred to as a distal side, and a side to opposite the distal side is referred to as a proximal side. In the illustrated embodiment, the core wire 2 is a single wire. Alternatively, the core wire 2 may be a bundle of plural wires. In addition, a coil may be wound around the outer circumference of the core wire 2. The core wire 2 has a distal-side wire section 2a provided at the distal end and a proximal-side wire section 2b provided on the proximal side of the distal-side wire section 2a. The proximal-side wire section 2b has a diameter larger than that of the distal-side wire section 2a. Although not shown, the diameter may be gradually increased from the distal-side wire section 2a to the proximal-side wire section 2b.

The core wire 2 is entirely made of Ni-Ti (nickel-titanium) alloy. Alternatively, the distal-side wire section 2a of the core wire 2 may be made of Ni-Ti alloy, and the second wire section may be made of stainless steel (SUS) material.

The coil element 3 is wound around the longitudinal axis 101 of the core wire 2. The coil element 3 may be provided only at the distal end of the core wire 2 as illustrated, or may be provided throughout the longitudinal direction of the separator 1. The coil element 3 has a first coil unit 4 and a second coil unit 5. The boundary between the first coil unit 4 and the second coil unit 5 is defined by a distal joint part 13 and a proximal joint part 14 of the second coil unit 5 as described later.

The first coil unit 4 has a constant outer diameter along the longitudinal direction. In this embodiment, the first coil unit 4 has a distal part 4a (or first part) and a proximal part 4b (or second part) which are longitudinally separated from each other. In the first coil unit 4, adjacent turns (4c, 4c) of wire material forming the coil are made in close contact with each other to form a close wound part. The first coil unit 4 is separated from the core wire 2 by a predetermined distance. The distal part 4a of the first coil unit 4 has a distal end 4d joined to the core wire 2 using solder and forming a joint part 6. The proximal part 4b of the first coil unit 4 has a proximal end 4e joined to the core wire 2 using solder and forming a joint part 7. The first coil unit 4 is fixed to the core wire 2 via the joint parts 6, 7, and the remaining part is separated from the core wire 2 by a predetermined distance, and a hollow part 4f is formed between the first coil unit 4 and the core wire 2. As described above, although the first coil unit 4 is joined to the core wire 2 at two places that are the joint parts 6, 7, the first coil unit 4 may be joined to the core wire 2 at three or more places.

The second coil unit 5 is provided between the distal part 4a and the proximal part 4b of the first coil unit 4. The second coil unit 5 has a close wound part at least partially in the longitudinal direction. In the close wound part, adjacent turns (5a, 5a) of wire material constituting the second coil unit 5, are made in close contact with each other. In the second coil unit 5, an open wound part in which adjacent turns are not made in close contact with each other is provided in a part where the close wound part is not provided. As illustrated, the close wound part may be provided throughout the second coil unit 5 in the longitudinal direction. As shown in Fig. 1, a winding direction of the second coil unit 5 is inclined at a predetermined angle larger than zero degrees with respect to a direction (indicated by a numeral 102 in Fig. 6) perpendicular to the longitudinal direction. That is, a pitch angle is greater than zero. Please note that a dashed line shown with the numeral 102 is drawn so as to correspond to a position of a peak 10a as described later.

The second coil unit 5 has a distal front end (may be referred to as a first end or distal end) 8, a proximal rear end (may be referred to as a second end or proximal end) 9, and a bulging part 10 provided between the front end 8 and the rear end 9. In the separator 1, the bulging part 10 can mainly function as a thrombus crushing member. In this embodiment, the front end 8 and the bulging part 10 are connected by a distal extension 11, and the rear end 9 and the bulging part 10 are connected by a proximal extension 12, respectively.

At the front end 8 and the rear end 9 of the second coil unit 5, the distal joint part 13 and the proximal joint part 14 made of solder are provided. The distal joint part 13 is provided from the front end 8 of the second coil unit 5 to the distal part 4a of the first coil unit 4, and the proximal joint part 14 is provided from the rear end 9 of the second coil unit 5 to the proximal part 4b of the first coil unit 4. The second coil unit 5 is separated from the core wire 2 by a predetermined distance at parts other than the distal joint part 13 and the proximal joint part 14, and a hollow part 5b is formed between the second coil unit 5 and the core wire 2. As described above, although the two joint parts 13, 14 are provided in the second coil unit 5, three or more joint parts may be provided.

In this embodiment, the distal part 4a of the first coil unit 4 and the second coil unit 5 are provided as separate pieces, and the proximal part 4b of the first coil unit 4 and the second coil unit 5 are integrally provided. Although the distal part 4a and the proximal part 4b of the first coil unit 4, and the second coil unit 5 are shown to have the same strand diameter in the drawing, these parts may have different diameters. For example, in case where the strand diameter of the second coil unit 5 is larger than the strand diameter of the first coil unit 4, the bulging part 10 of the second coil unit 5 can function as a thrombus crushing member with better performance. The front end 8 of the second coil unit 5 and the distal part 4a of the first coil unit 4 are joined to each other via the distal joint part 13 of the second coil unit 5. The front end 8 of the second coil unit 5 and the distal part 4a of the first coil unit 4 may be also joined to the core wire 2 via the distal joint part 13 of the second coil unit 5. The rear end 9 of the second coil unit 5 and the proximal part 4b of the first coil unit 4 are joined to the core wire 2 via the proximal joint part 14 of the second coil unit 5.

The bulging part 10 has a function to crush or entwine with the foreign substance present in the blood vessel. In terms of firmly fixing the second coil unit 5, it would be preferable that the distal joint part 13 and the proximal joint part 14 are provided at positions closer to the bulging part 10. On the other hand, in this embodiment, the rear end of the second coil unit 5 provided with the proximal joint part 14 is arranged at a position slightly separated toward the proximal side from the bulging part 10, so as not to degrade the operability of the separator 1 at the curved portion of the blood vessel. In this case, the length (i.e., a longitudinal dimension) of the proximal extension 12 is larger than the length of the distal extension 11. With this configuration, as described later, even when the blood vessel in which the separator 1 is advanced is curved, the separator 1 can be favorably operated. This effect is particularly remarkable when the length of the proximal extension part 12 is about 1.5 to about 30 times as long as the length of the distal extension 11.

The bulging part 10 has the peak 10a at which the outer diameter of the second coil unit 5 is maximum. The bulging part 10 has an axisymmetric shape on the distal side and the proximal side with respect to the peak 10a. The number of peak 10a of the bulging part 10 is not limited to one. The bulging part 10 bulges radially outward with respect to the front end 8 and the rear end 9. In other words, the outer diameter (radial dimension) of the bulging part 10 is larger than outer diameters (radial dimension) of the front end 8 and the rear end 9. Further, the outer diameter of the second coil unit 5 smoothly increases from the front end 8 and the rear end 9 toward the bulging part 10. When the number of peak of the bulging part 10 is two or more, the outer diameter of the second coil unit 5 changes smoothly between the peaks.

The coil element 3 is made of material selected from SUS (stainless steel), gold, platinum, tungsten, and the like. Gold, platinum, and tungsten are examples of radiopaque materials. In the embodiment, an X-ray contrast marker is provided at the distal part 4a of the first coil unit 4 in order to improve visibility when the separator 1 is inserted into a deep location in the blood vessel. Further, the second coil unit 5 may be provided with the X-ray contrast marker. The second coil unit 5 has the bulging part 10 with a characteristic shape, and the visibility during radiographic visualization can be particularly improved by providing the second coil unit 5 with the X-ray contrast marker. In a specific example, the distal part 4a of the first coil unit 4 may be made of platinum. Furthermore, in order to improve joining property with solder, portions around the joint parts 6, 7, 13, 14 of the first coil unit 4 and the second coil unit 5 may be made of SUS. In the separator 1, instead of making at least a part of the coil element 3 with radiopaque material, the at least a part of the coil element 3 may be coated with radiopaque material to thereby provide an X-ray contrast marker at this part.

In an embodiment, the first coil unit 4 and the second coil unit 5 of the coil element 3 are made of SUS. In this embodiment, in order to improve visibility during radiographic visualization, the coil element 3 may include a third coil unit (not shown) which is made of platinum and wound around the longitudinal axis 101 of the core wire 2 radially inward of the first coil unit 4 and the second coil unit 5. The third coil unit may be provided, for example, around the distal joint part 13. In addition, the third coil unit may be wound around the core wire 2 while being in contact with the core wire 2.

### [4. Suction system]

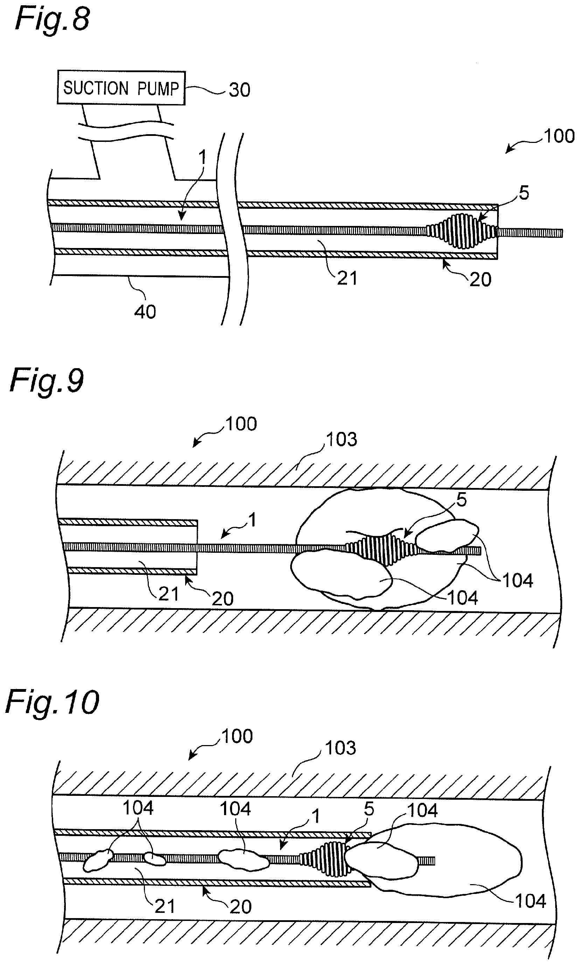

Fig. 8 is a view showing a suction system 100 provided with the separator 1 according to the embodiment of the present invention. The suction system 100 includes a catheter 20 and a suction pump 30, as well as the separator 1. The catheter 20 has a lumen 21 sized to allow the separator 1 to be inserted therethrough. The suction pump 30 is connected to the catheter 20 via a Y-connector 40.

Next, a method of using the suction system 100 is described below with reference to Figs. 9 to 11. When using the suction system 100, an operator operates the catheter 20 and the separator 1 while confirming the X-ray contrast marker provided at the coil element 3 of the separator 1 during radiographic visualization. The operator advances the catheter 20 and the separator 1, for example, from the femoral artery toward a part where a foreign substance 104, such as thrombus, is clogged in a cerebral blood vessel 103 and blood flow is blocked, while the separator 1 is kept inserted into the lumen 21 of the catheter 20.

After the catheter 20 and the separator 1 reach the part where the foreign substance 104 is clogged in the cerebral blood vessel 103, the operator projects the separator 1 out of the tip of the catheter 20 and further advances the separator 1 so that the distal end 4d of the first coil unit 4 is positioned behind the foreign substance 104. This operation can be easily performed by providing the X-ray contrast marker at the distal part 4a of the first coil unit 4 including the joint part 6. In this state, the operator finely moves the separator 1 back and forth in the longitudinal direction, so that particularly the bulging part 10 of the second coil unit 5 works, thereby the foreign substance 104 is crushed by or entwined with the coil element 3.

In this case, at the front end 8 and the rear end 9 of the second coil unit 5 there are respectively provided the distal joint part 13 and the proximal joint part 14 which are joined to the distal part 4a and the proximal part 4b of the first coil unit 4 using solder. Accordingly, the second coil unit 5 is rigidly fixed to at least one of the core wire 2 and the first coil unit 4. With this configuration, for example, even when the foreign substance 104 is firmly attached to a blood vessel wall of the cerebral blood vessel 103 or the foreign substance 104 is hardened and a force acting on the bulging part 10 of the second coil unit 5 from the foreign substance 104 in the longitudinal direction (proximal side) becomes larger when the separator 1 is moved back and forth, the second coil unit 5 is no longer separated from the first coil unit 4. In particular, because the outer diameter of the second coil unit 5 is smoothly enlarged from the front end 8 and the rear end 9 toward the bulging part 10, the bulging part 10 can be prevented from being trapped by the foreign substance 104 while the separator 1 is moved back and forth. Accordingly, the force acting on the bulging part 10 in the longitudinal direction (proximal side) from the foreign substance 104 can be reduced, and the stress applied to the joint parts 13, 14 can be also reduced. In addition, because the second coil unit 5 has the close wound part at least partially in the longitudinal direction, a portion having the close wound part can be prevented from being trapped by the foreign substance 104, and the force acting in the longitudinal direction (proximal side) from the foreign substance 104 can be reduced, and further, the stress applied to the joint parts 13, 14 can be also reduced.

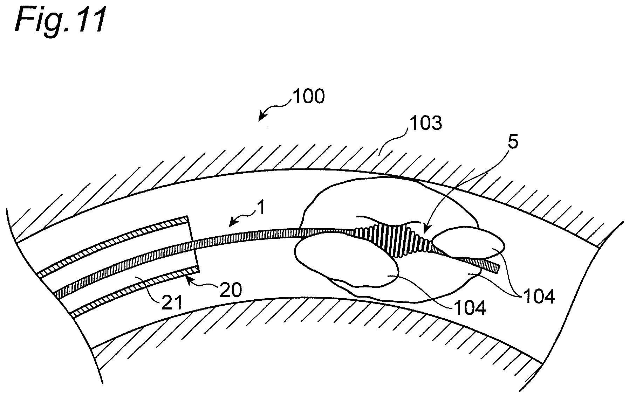

In addition, because the bulging part 10 is formed of a coil, for example, even when the foreign substance 104 is positioned in the curved portion of the cerebral blood vessel 103 (see Fig. 11), it is easier for the separator 1 to move back and forth in the curved portion. This can reduce a risk that the operator erroneously operates the separator 1 to damage the cerebral blood vessel 103. In this case when the bulging part 10 hits the foreign substance 104 in the curved portion of the cerebral blood vessel 103, a force may act such that the separator 1 curved along the cerebral blood vessel 103 is further curved at the proximal part of the bulging part 10. Also in this case, since the rear end 9 of the second coil unit 5 provided with the proximal joint part 14 is located at a position slightly separated from the bulging part 10 toward the proximal side, the proximal part of the bulging part 10 in the separator 1 can be further curved without being obstructed by the proximal joint part 14, hence, the operation of the separator 1 can be favorably performed in the curved portion of the blood vessel.

Thereafter, the operator advances only the catheter 20 to accommodate the second coil unit 5 of the coil element 3 of the separator 1 in the lumen 21 of the catheter 20. As a result, the foreign substance 104 crushed or entwined enters the lumen 21, while following the second coil unit 5. This operation can be easily performed particularly when the second coil unit 5 is provided with the X-ray contrast marker. Then, after operating the suction pump 30, the foreign substance 104 can move toward the proximal side in the lumen 21 of the catheter 20 and be removed outside of the body.

### [Other embodiments]

It should be understood that the present invention is not limited to the content of the above-mentioned embodiment. Also, the features of the plural embodiments described in the present description may be freely combined. In addition, various improvements, design changes and deletions may be applied to each embodiment.

For example, in the above-mentioned embodiment, the separator 1 is used to remove the thrombus and the like in the cerebral blood vessel outside the body. The present invention is not limited to this, and in another embodiment, the separator 1 may be used to remove the thrombus or the like present in other blood vessels such as coronary artery.

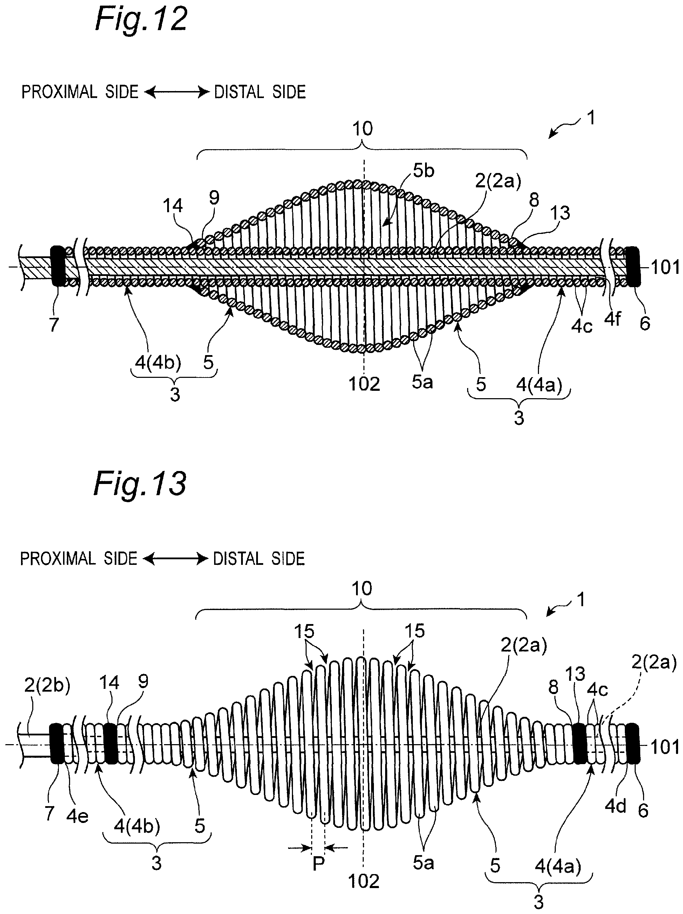

Further, in the above-mentioned embodiment, the first coil unit 4 includes the distal part 4a (or first part) and the proximal part 4b (or second part) which are longitudinally separated from each other at the bulging part 10, and the joint parts 13, 14 are provided at the front end 8 and the rear end 9 of the second coil unit 5, respectively. The present invention is not limited to this, and in another embodiment, as shown in Fig. 12, the first coil unit 4 may be continuously provided also in the part of the bulging part 10 of the second coil unit part 5. In this embodiment, the front end 8 and the rear end 9 of the second coil unit 5 may be joined to the distal part 4a and the proximal part 4b of the first coil unit 4 using, for example, a UV adhesive or solder to provide the joint parts 13, 14.

Moreover, in the above-mentioned embodiment, the second coil unit 5 is at least partially provided with the close wound part. The present invention is not limited to this, and in another embodiment, as shown in Fig. 13, an open wound part may be provided in which adjacent turns are not made in close contact with each other throughout the entire second coil unit 5. In the open wound part, the adjacent turns (4c, 4c) of wire material forming the coil are not made in close contact with each other, and are wound at a predetermined pitch P. In the second coil unit 5 provided with the open wound part, a gap 15 is formed between the adjacent turns. When the separator 1 shown in Fig. 13 is finely moved back and forth in the longitudinal direction in the cerebral blood vessel 103 in a state in which the foreign substance 104 is clogged and blood flow is blocked, an effect can be obtained that the foreign substance 104 is more easily entwined therewith.

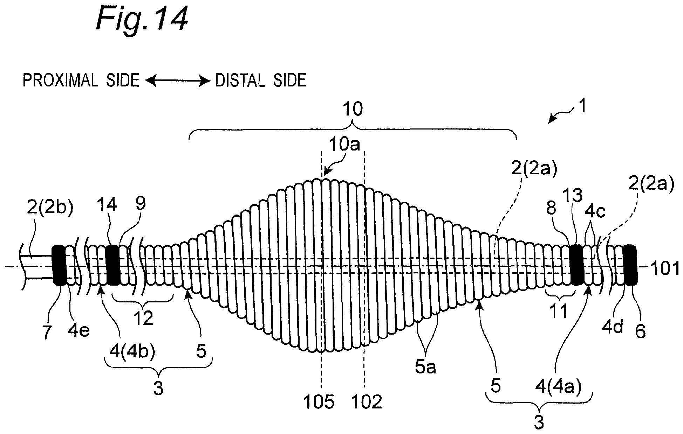

Moreover, in the above-mentioned embodiment, there is provided the bulging part 10 having the axisymmetric shape on the distal side and the proximal side with respect to the peak 10a. The present invention is not limited to this, and in another embodiment, as shown in Fig. 14, the bulging part 10 may have an asymmetrical shape on the distal side and the proximal side with respect to the peak 10a. The position of the peak 10a shown in Fig. 14 (corresponding to a dashed line 105) is located on the proximal side of the position of the peak 10a shown in Fig. 6 (corresponding to the dashed line 102). In this embodiment, because a curve extending from the front end 8 of the second coil unit 5 to the peak 10a can be made smooth, resistance acting when crushing or entwining with the foreign substance 104 in the coil element 3 including the bulging part 10 can be reduced, and the stress applied to the joint parts 13, 14 can be also reduced.

In the above-mentioned embodiment, the distal part 4a of the first coil unit 4 and the second coil unit 5 are provided as separate pieces, and the proximal part 4b of the first coil unit 4 and the second coil unit 5 are integrally provided. The present invention is not limited to this, and in another embodiment, the distal part 4a of the first coil unit 4 and the second coil unit 5 may be integrally provided. In this case, the entire coil element 3 is integrally provided. In addition, the proximal part 4b of the first coil unit 4 and the second coil unit 5 may be provided as separate pieces.

Moreover, in the above-mentioned embodiment, the joint parts 6, 7, 13, 14 are formed of solder. The present invention is not limited to this, and in another embodiment, as a joining material, a sinterable metal joining material may be used instead of solder, or the joint parts 6, 7, 13, 14 may be formed by welding without using the joining material.

### [EXPLANATORY NOTE]

- 50: CATHETER
- 51: TIP
- 52: X-RAY CONTRAST MARKER
- 53, 54, 55: TUBE
- 56: STRAIN RELIEF
- 57: HUB
- 61: INNER LAYER MEMBER
- 61a: INNER CIRCUMFERENTIAL SURFACE
- 62: OUTER LAYER MEMBER
- 62a: OUTER CIRCUMFERENTIAL SURFACE
- 71: FIRST REINFORCING MEMBER
- 72: SECOND REINFORCING MEMBER
- 73: FIBER
- SA: TIP END SECTION
- SB: INTERMEDIATE SECTION
- SC: BASE END SECTION
- 1: SEPARATOR
- 2, 2a, 2b: CORE WIRE
- 3: COIL ELEMENT
- 4: FIRST COIL UNIT
- 4a: DISTAL PART (OF FIRST COIL UNIT)
- 4b: PROXIMAL PART (OF FIRST COIL UNIT)
- 5: SECOND COIL UNIT
- 6, 7: JOINT PART
- 8: FRONT END (OF SECOND COIL UNIT)
- 9: REAR END (OF SECOND COIL UNIT)
- 10: BULGING PART
- 11: DISTAL EXTENSION
- 12: PROXIMAL EXTENSION
- 13: DISTAL JOINT PART
- 14: PROXIMAL JOINT PART
- 20: CATHETER
- 21: LUMEN
- 30: SUCTION PUMP
- 40: Y-CONNECTOR
- 101: LONGITUDINAL AXIS (OF CORE WIRE)
- 103: CEREBRAL BLOOD VESSEL
- 104: FOREIGN SUBSTANCE

## Claims

1. A catheter having an elongated lumen extending along a longitudinal axis, the catheter comprising:
an inner layer member having an inner circumferential surface in contact with the lumen;
an outer layer member having an outer circumferential surface of the catheter;
a first reinforcing member having a shape of coil, provided outside the inner layer member, between the inner circumferential surface and the outer circumferential surface; and
a second reinforcing member having a shape of braid, provided outside the first reinforcing member, between the inner circumferential surface and the outer circumferential surface.

2. The catheter according to Claim 1, wherein the second reinforcing member is constituted of an elongated member made of synthetic resin.

3. The catheter according to Claim 1 or 2, wherein the elongated member is constituted of a bundle of plural fibers.

4. The catheter according to any one of Claims 1 to 3, wherein the first reinforcing member is constituted of a wire having a rectangular cross section.

5. The catheter according to any one of Claims 1 to 4, wherein the first reinforcing member is constituted of a metal wire.

6. The catheter according to any one of Claims 1 to 5, wherein the second reinforcing member is provided in a region at a predetermined distance from the proximal end.

7. A suction system comprising:
the catheter according to any one of Claims 1 to 6; and
a suction pump connected to the catheter.

8. A separator comprising:
a core wire having a longitudinal axis; and
a coil element wound around the longitudinal axis of the core wire and having a distal end and a proximal end joined to the core wire;
wherein the coil element includes a first coil unit, a second coil unit having a first end and a second end connected to the first coil unit, and a distal joint part and a proximal joint part provided at the first end and the second end, respectively, and
the second coil unit has a bulging part having an outer diameter larger than outer diameters of the first end and the second end.

9. The separator according to Claim 8, wherein the second coil unit has a distal extension and a proximal extension which connect the first end and the second end with the bulging part, respectively, and
the length of the proximal extension is larger than the length of the distal extension.

10. The separator according to Claim 8 or 9, wherein the outer diameter of the second coil unit smoothly increases from the first end and the second end toward the bulging part.

11. The separator according to any one of Claims 8 to 10, wherein the strand diameter of the second coil unit is larger than the strand diameter of the first coil unit.

12. The separator according to any one of Claims 8 to 11, wherein the first coil unit has a first part and a second part which are longitudinally separated from each other, and
the first end and the second end of the second coil unit are joined to the first part and the second part of the first coil unit, respectively.

13. A suction system comprising:
the separator according to any one of Claims 8 to 12;
a catheter having a lumen through which the separator is inserted; and
a suction pump connected to the catheter.
